(19) Europäisches Patentamt
European Patent Office
Office européen des brevets

(11) **EP 2 653 205 B1**

(12) **EUROPÄISCHE PATENTSCHRIFT**

(45) Veröffentlichungstag und Bekanntmachung des Hinweises auf die Patenterteilung:
**10.01.2018 Patentblatt 2018/02**

(51) Int Cl.:
*B01D 19/04* (2006.01)  *C09D 7/12* (0000.00)
*C07C 69/34* (2006.01)  *C07C 69/50* (2006.01)
*C08G 65/48* (2006.01)  *C08G 65/34* (2006.01)

(21) Anmeldenummer: **13162853.9**

(22) Anmeldetag: **09.04.2013**

(54) **Entschäumer und Verwendung von Polyglycerinpartialestern als Entschäumer**

Antifoaming agents and use of polyglycerine partial esters as antifoaming agents

Agent antimousse et utilisation d'esters partiels du polyglycérol comme agent antimousse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.04.2012 DE 102012206574**

(43) Veröffentlichungstag der Anmeldung:
**23.10.2013 Patentblatt 2013/43**

(73) Patentinhaber: **Evonik Degussa GmbH 45128 Essen (DE)**

(72) Erfinder:
• **Nilewski, Margitta**
 **45359 Essen (DE)**
• **Favresse, Philippe**
 **40880 Ratingen (DE)**
• **Scharf, André**
 **45355 Essen (DE)**
• **Gehrmann, Petra**
 **46238 Bottrop (DE)**
• **Mettin, Thomas**
 **42329 Wuppertal (DE)**
• **Schwan, Michael**
 **51375 Leverkusen (DE)**
• **Springer, Oliver**
 **46485 Wesel (DE)**
• **Wied, Tobias**
 **42109 Wuppertal (DE)**

(56) Entgegenhaltungen:
**EP-A1- 0 835 862       EP-A1- 2 360 140
WO-A1-2011/100420   WO-A1-2012/007754
DE-A1- 19 641 076     DE-A1- 19 835 968
JP-A- H09 131 503     JP-A- 2000 212 483
US-A- 2 284 127        US-A- 3 413 081
US-A- 5 326 499        US-A- 5 429 718**

EP 2 653 205 B1

**Beschreibung**

Die vorliegende Erfindung betrifft die Verwendung von Polyglycerinpartialestern als Entschäumer, insbesondere in Lack- und Farbanwendungen, Entschäumer enthaltend Polyglycerinpartialester sowie entsprechende Polyglycerinpartialester.

Die Produktklasse der Entschäumer ist bereits seit mehreren Jahrzehnten in vielen Produktbereichen im Einsatz. Beispiele für diese Produktbereiche sind Lebensmittel, Pharma, Kosmetik, Papier, Farben und Lacke.

Entschäumer bewirken die beschleunigte Koaleszenz von Gasblasen in fluiden Stoffsystemen. Hierdurch wird die Schaumbildung vermieden bzw. deutlich reduziert. Sofern vor Zugabe der Entschäumer bereits Schaumanteile vorhanden sind, werden diese destabilisiert.

Viele bekannte Entschäumer wie sie in den US 6,605,183, US 5,914,362, US 5,846,454 und US 4,690,713 eingeführt werden, basieren auf einer Kombination von Silikonöl oder Silica Partikeln. In einer Vielzahl von Stoffsystemen gilt es allerdings Silikonöl oder Silica Partikel zu vermeiden, da durch diese Komponenten andere maßgebliche Funktionalitäten gestört werden. Sogenannte wasserbasierende Entschäumer, welche aus in Wasser verteiltem Öl (Öl in Wasser Emulsionen) bestehen, wie sie beispielhaft von der US 4,976,888 und der US 5,429,718 beschrieben werden, finden ebenfalls seit den 50er Jahren des 20. Jahrhunderts Einsatz in industriellen Anwendungen.

Eine weitere Gruppe von Entschäumern kombiniert ethoxylierte Polyether mit tensidischer Struktur mit polyhydrischen Alkohol-Fettsäure-estern. Ein Beispiel hierzu wird in US 5,346,511 beschrieben.

Die Wirkung von Entschäumern wird entscheidend von ihren grenzflächenaktiven Eigenschaften bestimmt, insbesondere von der Fähigkeit sich an der Phasengrenzfläche zwischen unterschiedlichen fluiden Medien anzureichern.

Entschäumende Substanzen, die durch die Vernetzung von Glykolethereinheiten mittels Isocyanaten oder Dicarbonsäuren entstehen, werden in DE 44 04 202 beschrieben.

Entschäumende Mischungen aus Sojaöl, Mineralöl, fein verteiltem Siliciumdioxid, Fettsäuremonoestern des Glycerins und Fettsäuremonoestern von polyalkoxyliertem Sorbitan werden in EP 0 765 5811 beschrieben.

In EP 450605 werden Fettsäure modifizierte Diglyceride als Teilkomponente eines Entschäumers für Lebensmittel beschrieben. WO 00/051708 beschreibt eine wässrige Entschäumeremulsion, deren Wirkstoff auf einem mit Fettsäuren modifizierten Polyester beruht.

Die Verwendung von mit Fettsäure modifizierten und mit Dicarbonsäure vernetzten Glyzerinen als Emulgatoren ist in DE 25 17 354 beschrieben.

Die Verwendung von partialveresterten (bis 90 %) Polyglycerinen als Tenside wird in JP 2000-230191 A beschrieben.

Im EP 0 403 913 A1 werden gegebenenfalls ethoxylierte und/oder propoxylierte Oligoglyzerine beschrieben, die gegebenenfalls mit hydroxysubstituierten C2- bis C 18 Fettsäuren modifiziert werden und als Pigmentdispergatoren für wässrige Lackdispersionen eingesetzt werden können.

Die JP 2011-083715 A beschreibt Polyglyzerinfettsäureester als Teilkomponente eines aus mehreren Komponenten aufgebauten Entschäumers.

EP 0 878 224 beschreibt entschäumend wirkende Substanzen, die aus partialveresterten Oligoglyzerinen bestehen, die zusätzlich alkoxyliert sind und mit organischen oder anorganischen Feststoffen abgemischt werden.

DE 196 41 076 A1 beschreibt einen Entschäumer auf Basis von Mono- oder Diglyceriden für den Einsatz in der Papierindustrie.

DE 198 35 968 A1 beschreibt einen Entschäumer bestehend aus Polyglycerinester auf Basis von Monocarbonsäuren.

WO 2011/100420 A1 offenbart eine Zusammensetzung für die Textil- und Gewebebehandlung enthaltend Polyglycerinester.

Die Aufgabe der vorliegenden Erfindung ist die Bereitstellung von verbesserten Entschäumern, die an die jeweiligen vorliegenden Eigenschaften des Anwendungslacksystems angepasst werden können.

Die Wirkungsweise von Entschäumern ist von der Fähigkeit abhängig, in Wechselwirkungen an der Phasengrenze zwischen eingeschlossenen Luftblasen und der umgebenden flüssigen Phase einzugreifen.

Um in diese Wechselwirkungen eingreifen zu können, ist ein Gleichgewicht zwischen Polarität, Viskosität und generellen grenzflächenaktiven Eigenschaften auf Seiten des Entschäumers notwendig.

Häufig zieht die Einstellung dieser Charakteristika zur Erreichung einer guten entschäumenden Wirkung, gleichzeitig die negative Beeinflussung weiterer Lackeigenschaften wie z. B. des Verlaufes oder der allgemeinen Oberflächenqualität des Lackfilmes nach sich.

Dieselben Charakteristika des Entschäumers, die zu einer Vermeidung/Reduzierung der Schaumbelastung führen, verursachen somit gleichzeitig auch eine Beeinträchtigung anderer wichtiger Lackeigenschaften.

Als Beispiele für Substanzen, welche gleichzeitig Schaum verhindernde und die Lackoberfläche beeinträchtigende Eigenschaften haben, sind die Klassen der Silikonöle bzw. der Polyethersiloxane zu nennen.

Die zu lösende Aufgabe der vorliegenden Erfindung besteht daher darin, die Verhinderung/Reduzierung der Schaumbildung in Lacksystemen zu ermöglichen, und gleichzeitig sonstige Lackeigenschaften wie z. B. die Oberflächengüte nicht negativ zu beeinflussen.

Die genannte Aufgabe wird durch die Verwendung spezifisch ausgewählter Polyglycerinester als Entschäumer gelöst.

Gegenstand der vorliegenden Erfindung ist somit die Verwendung von Polyglycerinpartialestern von gesättigten oder ungesättigten, linearen oder verzweigten Fettsäuren und/oder aromatischen Monocarbonsäuren und mehrfunktionellen Carbonsäuren, die erhältlich sind durch Veresterung eines Polyglyceringemisches mit gesättigten oder ungesättigten, linearen oder verzweigten Fettsäuren mit 4 bis 22 C-Atomen und/oder aromatischen Monocarbonsäuren mit 7 bis 22 C-Atomen und mehrfunktionellen Carbonsäuren mit 4 bis 54 C-Atomen und/oder deren Anhydriden oder Ester als Entschäumer, wobei der Veresterungsgrad des Polyglycerins zwischen 76 und 100 % liegt, und dass die Polyglyceringemische auf Polyglycerinen mit einem mittleren Kondensationsgrad von 3-5 basieren.

Die erfindungsgemäßen Polyglycerinpartialester unterscheiden sich von denen aus dem Stand der Technik, zum Beispiel denen in JP 2011-083715, dahingehend, dass eine zusätzliche Vernetzung mittels Dicarbonsäuren zwischen den Polyglycerineinheiten hergestellt wird, um die Polydispersität an die zu lösende Aufgabe anzupassen.

So hat sich überraschend gezeigt, dass sich spezifische mit Fettsäure und/oder aromatischen Monocarbonsäuren modifizierte Polyglycerine, bei denen eine Vernetzung durch mehrfunktionelle Carbonsäuren, beispielsweise Dicarbonsäuren bzw. Dicarbonsäureanhydride vorliegt, besonders gut als Entschäumer eignen. Durch die, über die Fettsäuremodifikation der Polyglycerine hinausgehende, Vernetzung der Polyglycerinoligomere, werden zusätzliche Freiheitsgrade bei der Synthese der Entschäumermoleküle gewonnen. Hierdurch können verschiedene physikalische bzw. physiko-chemische Parameter der entstehenden Moleküle, beispielsweise Viskosität und/oder Polarität, breiter beeinflusst werden, als dies bisher der Fall ist. Durch die solcherart vorgenommene Optimierung von z. B. Molmasse, Viskosität oder Polarität, kann ein besondere entschäumende Wirkung erzielt werden.

Entsprechende Polyglycerinpartialester, die erfindungsgemäß als Entschäumer eingesetzt werden, sind dem Fachmann als solches bekannt, beispielsweise aus EP 0 835 862. Insbesondere bevorzugt weisen die eingesetzten bzw. erfindungsgemäßen Polyglycerinpartialester einen Veresterungsgrad des Polyglycerins zwischen 80 und 100 % und ganz besonders bevorzugt zwischen 91 und 100 % auf. Derartige Polyglycerinpartialester sind aus dem Stand der Technik, beispielsweise aus EP 0 835 862, nicht bekannt.

Die Bestimmung des Veresterungsgrades erfolgt über eine volumetrische Ermittlung der Säurezahl (SZ). Die Säurezahl gibt die Masse an mg KOH an, die notwendig ist, um die in 1 g Produkt enthaltenen freien Säuren zu neutralisieren. Die Säurezahl kann mittels der hier beschriebenen Methode bestimmt werden. Die beschriebene Methode wurde in Anlehnung an DGF C-V 2, Ph.EUR. 2.5.1, ISO 3682, ASTM D 974, DIN EN ISO 2114 erstellt.

Zur Bestimmung der Säurezahl wird die Probe in einem geeigneten Lösungsmittel gelöst und im Anschluss werden die anwesenden freien Säuren mit Kalilauge titriert.

Als Lösungsmittel wird eine Mischung aus Ethanol und Toluol im Verhältnis 1 : 1 verwendet. Andere mögliche Lösungsmittel sind z. B. Ethanol oder Isopropanol, wobei die alternativen Lösungsmittel keinen Einfluss auf das Ergebnis der Messmethode haben. Die verwendeten Lösungsmittel bzw. Lösungsmittelgemische sind lediglich im Vorfeld gegen Phenolphthalein zu neutralisieren um eine Verfälschung des Analysenergebnisses zu vermeiden.

Als Titrant kommt unter anderem 0,5 N Kalilauge, 0,1 N Kalilauge bzw. 0,02 N Kalilauge in Frage. Als Lösungsmittel für die Kalilauge kommt Wasser oder Ethanol in Frage.

Als Indikator für das Erreichen des Umschlagpunktes kann z. B. mit einer 1% igen Phenolphthaleinlösung in Ethanol gearbeitet werden.

Zur eigentlichen Durchführung wird die zu untersuchende Probe auf 0,1 % genau eingewogen. Im Anschluss werden ca. 50-100 ml des neutralisierten Lösungsmittels hinzugegeben und die Probe, sofern notwendig, unter leichtem Erwärmen gelöst.

Nach Zusatz der Phenolphthaleinlösung wird mit eingestellter Kalilauge bis zum bleibenden Farbumschlag titriert. Zur Auswertung wird die folgende Berechnungsformel verwendet:

$$\text{Säurezahl [mg KOH/g]} = \frac{V \times M_{KOH} \times N}{E}$$

Legende:

V = Verbrauch an Titrant [ml]
N = Normalität Titrant
E = Einwaage Probe [g]

$M_{KOH}$ = Molare Masse von KOH

Dabei gilt die Beziehung, dass mit sinkender Säurezahl der Veresterungsgrad zunimmt. Unter Berücksichtigung der eingesetzten Stoffmenge an Carboxylgruppen tragenden Molekülen sowie der eingesetzten Stoffmenge an Hydroxylgruppen tragenden Molekülen, kann mittels der Säurezahl auf den Veresterungsgrad geschlossen werden.

Die Säurezahl steht für die noch verbliebenen Carboxylgruppen des Produktes, und erlaubt somit einen Rückschluss auf den prozentualen Anteil an abreagierten Carboxylgruppen der ursprünglichen Reaktanden.

Da im Rahmen des Veresterungsschrittes für jede reagierende Carboxylgruppe auch eine Hydroxylgruppe reagiert, ist die Anzahl der abreagierten Hydroxylgruppen gleich der Anzahl der abreagierten Carboxylgruppen. Der prozentuale Anteil an abreagierten Hydroxylgruppen der ursprünglichen Reaktanden kann schließlich mit dem Veresterungsgrad gleichgesetzt werden.

Die erfindungsgemäß eingesetzt bzw. erfindungsgemäßen Polyglycerinpartialester werden durch Veresterung und Vernetzung von mindestens drei Komponenten erhalten.

Grundlage für die Polyglycerinpartialester sind die eingesetzten Polyglyceringemische, die insbesondere auf Polyglycerinen mit einem mittleren Kondensationsgrad von 3-5, das heißt mit 3 - 5 Wiederholungseinheiten, beruhen. Dabei handelt es sich um technische Polyglycerinmischungen, die z. B. durch alkalisch katalysierte Kondensation von Glycerin bei erhöhten Temperaturen erhalten werden, aus denen sich gegebenenfalls durch Destillationsverfahren Fraktionen mit dem erwünschten Kondensationsgrad erhalten lassen. Ebenfalls geeignet sind auch Polyglycerine, die auf anderem Wege, z. B. aus Epichlorhydrin oder Glycidol gewonnen werden. Besonders geeignete Polyglycerine weisen folgende Oligomerenverteilung auf (in Klammern angegeben sind bevorzugte Bereiche):

Glycerin: 0,01 Gew.-% bis 20 Gew.-% (3 Gew.-% bis 12 Gew.-%),
Diglycerine: 0,01 Gew.-% bis 60 Gew.-% (20 Gew.-% bis 40 Gew.-%),
Triglycerine: 0,01 Gew.-% bis 60 Gew.-% (15 Gew.-% bis 35 Gew.-%),
Tetraglycerine: 0,01 Gew.-% bis 30 Gew.-% (5 Gew.-% bis 20 Gew.-%),
Pentaglycerine: 0,01 Gew.-% bis 20 Gew.-% (0,1 Gew.-% bis 15 Gew.-%) und
Oligoglycerine: ad 100 Gew.-%,

wobei sich die angegebenen Gewichtsprozente auf die Gesamtmenge an eingesetztem Polyglycerin beziehen und diese Verteilung mit der GC-Methode wie unten ausgeführt bestimmt wird.

Dem Fachmann ist bewusst, dass Polyglycerin aufgrund seiner polymeren Eigenschaft eine statistische Mischung verschiedener Verbindungen darstellt. Polyglycerin kann ausgebildete Etherbindungen zwischen zwei primären, einer primären und einer sekundären oder zwei sekundären Positionen der Glycerinmonomere aufweisen; ebenfalls sind zyklische Strukturen mit einem oder mehreren Ringen bekannt. Für Details siehe z. B. "Original synthesis of linear, branched and cyclic oligoglycerol standards", Cassel et al., Eur. J. Org. Chem. 2001, 875-896.

Eine geeignete GC Methode zur Bestimmung der Homologenverteilung beinhaltet die Hydrolyse oder Alkoholyse des erfindungsgemäßen (Poly-)glycerinpartialesters, Trennung des Polyglycerins von den entstandenen Säuren und Analyse durch Gaschromatographie.

Hierzu werden 0,6 g des erfindungsgemäßen (Poly-)glycerinpartialesters in 25 ml einer ethanolischen 0,5 M KOH Lösung unter Rückfluss für 30 Minuten gekocht und der pH mit Schwefelsäure auf pH 2-3 eingestellt. Die entstandenen Fettsäuren werden durch dreimalige Extraktion mit jeweils einem Volumen Petrolether abgetrennt. Die vereinigten Extrakte werden durch Evaporation auf ca. 10 ml eingeengt.

Ein 0,5 ml Probe wird in einem Autosamplergefäß mit 0,5 ml MTBE und 1 ml Trimethylanilinium Hydroxide (0.2 M in Methanol) versetzt und mit GC analysiert. Diese wird in einem Gaschromatographen, welcher mit einem split/splitless Injektor, einer Kapillarsäule und einem Flammenionisationdetektor ausgestattet ist, unter folgenden Bedingungen durchgeführt:

| | |
|---|---|
| Injektor | 290 °C, Split 30 ml |
| Injektionsvolumen: | 1 μl |
| Säule: | 30 m *0,32 mm HP1 0,25 μm |
| Trägergas: | Helium, head pressure 70 kPa |
| Temperaturprogramm: | 80 °C - 300 °C mit 8 °C/min, dann 20 |
| | Minuten bei 300 °C konditionieren. |
| Detektor: | FID bei          320 °C |
| | Wasserstoff     35 ml/min |
| | Luft            240 ml/min |
| | Make Up Gas     12 ml/min |

Hierdurch werden die Fettsäuren als ihre Methylester nach ihrer Kohlenstoffkettenlänge getrennt. Der relative Gehalt der einzelnen Fettsäuren kann durch Auswertung der Peakflächen bestimmt werden.

Der mit Petrolether extrahierte Rückstand wird mit Bariumhydroxid auf pH 7 bis 8 eingestellt, das ausgefallene Bariumsulfat durch Zentrifugation abgetrennt.

Der Überstand wird abgenommen und der Rückstand wird dreimal mit 20 ml Ethanol extrahiert.

Die vereinigten Überstände werden bei 80 °C und 50 mbar eingeengt, der Rückstand in Pyridin aufgenommen. Ein 0,5 ml Probe wird in einem Autosamplergefäß mit 1 ml N-Methyl-N-trifluoroacetamid versetzt und für 30 Minuten bei 80 °C erhitzt.

Das Polyglycerin wird als sein Trimethylsilyl-Derivat mit GC analysiert, wobei ein Gas-Flüssig-Chromatograph mit einem On-column Injektor und Flammenionisationdetektor unter den folgenden Bedingungen eingesetzt wird:

| | |
|---|---|
| Injektor: | On-column, oven tray |
| Injektionsvolumen: | 0.1 $\mu$l |
| Trägergas: | 3 ml/min Wasserstoff (constant flow) |
| Säule: | SimDist 12 m x 0,32 mm x 0,1 $\mu$m (Varian) |
| Temperaturprogramm: | 65 °C - 365 °C, 10 °C/min; dann 15 Minuten bei 365 °C konditionieren. |
| Detektor (FID): | 375 °C |

Unter diesen Bedingungen wird das Polyglycerin nach dem Polyymerisierungsgrad getrennt; zusätzlich können zyklische von linearen Isomeren bis zu einem Polymerisierungsgrad von fünf getrennt werden. Die Peakflächen der einzelnen Oligomere werden durch eine Lotrechte an der tiefsten Stelle zwischen den Peaks voneinander getrennt. Da die Auflösung für Oligomere, die einen höheren Polymerisationsgrad als sechs aufweisen, gering ist, werden die Peakflächen für Heptaglycerin und höhere Oligomere zusammengefasst und für die Berechnung des Polydispersitätsindexes als Heptaglycerin betrachtet. Zur Berechnung des Polydispersitätsindexes werden außerdem zyklische und lineare Isomere zusammengefasst.

Der relative Gehalt der einzelnen Oligomere/Isomere kann durch Auswertung der Peakflächen bestimmt werden.

In analoger Weise kann dieses Verfahren auch genutzt werden, um die eingesetzten Rohstoffe, welche zur Herstellung der erfindungsgemäßen Ester eingesetzt werden, zu charakterisieren.

Im Rahmen der vorliegenden Erfindung werden unter "Säuren" stets auch deren Derivate, insbesondere Anhydride oder Ester verstanden, das heißt anstelle der freien Säuren können auch Ester und sofern möglich auch Anhydride eingesetzt werden.

Weitere wesentliche Komponenten der erfindungsgemäß eingesetzten bzw. erfindungsgemäßen Polyglycerinpartialester sind die gesättigten oder ungesättigten, linearen oder verzweigten Fettsäuren mit 4 bis 22 C-Atomen, insbesondere mit 8 bis 22 C-Atomen bzw. deren Anhydride oder Ester. Hierzu eignen sich grundsätzlich alle dem Fachmann bekannten Fettsäuren dieser Art.

Als gesättigte lineare Fettsäurekomponenten eignen sich vor allem Laurinsäure, Tridecansäure, Myristinsäure, Palmitinsäure, Margarinsäure, Stearinsäure, Arachinsäure und Behensäure sowie Mischungen hieraus. Natürlich vorkommende Mischungen sind beispielsweise die Kokosfettsäuren, die als Hauptbestandteil Laurinsäure, daneben noch gesättigte $C_{14}$- bis $C_{18}$-Fettsäuren und gegebenenfalls in geringen Mengen gesättigte $C_{18}$- bis $C_{10}$-Fettsäuren und ungesättigte Fettsäuren enthalten, sowie Talgfettsäuren, welche im Wesentlichen eine Mischung aus Palmitinsäure und Stearinsäure darstellen.

Als gesättigte verzweigte Fettsäurekomponenten eignen sich vor allem Isostearinsäure und Phytansäure. Ein weiteres Beispiel für eine gesättigte verzweigte Fettsäurekomponente ist Isovaleriansäure.

Als ungesättigte lineare Fettsäurekomponenten eignen sich beispielsweise monoolefinisch ungesättigte Säuren, beispielsweise Hexadecensäuren, Octadecensäuren, wie Ölsäure (cis-9-Octadecensäure) oder Elaidinsäure (trans-9-Octadecensäure), Eicosensäuren und Docosensäuren, wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren, wie Linolsäure und Linolensäure sowie Mischungen hieraus. Besonders geeignet sind die flüssigen Fettsäuren, Öl-, Ricinol-, Eruca- und Isostearinsäure, die 18 bis 22 Kohlenstoffatome enthalten. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.

Als ungesättigte verzweigte Fettsäurekomponenten eignen sich beispielsweise 8-Methyl-trans-6-nonenosäure (Thiele R et al., J Agric Food Chem 2008, 56, 4219) oder 11-Methyloctadec-12-ensäure (Spencer GF et al., Lipids 1979, 14, 72).

Besonders bevorzugt ist die gesättigte oder ungesättigte, lineare oder verzweigte Fettsäure mit 4 bis 22 C-Atomen, insbesondere 8 bis 22 C-Atomen, ausgewählt aus Isostearinsäure, Ölsäure und/oder Linolensäure.

Als aromatische Monocarbonsäuren mit 7 bis 22 C-Atomen eignen sich insbesondere Benzoesäure, Phenylethansäure, 4-Decyloxybenzoesäure,
und/oder 4-Octadecyloxybenzoesäure bzw. deren Anhydride oder Ester, wobei Benzoesäure insbesondere bevorzugt ist.

Weitere wesentliche Komponenten der erfindungsgemäß eingesetzten bzw. erfindungsgemäßen Polyglycerinpartialester sind die mehrfunktionellen Carbonsäuren mit 4 bis 54 C-Atomen. Unter dem Begriff "mehrfunktionelle Carbonsäuren" im Sinne der vorliegenden Erfindung sind Carbonsäuren zu verstehen, die mehr als eine Carboxylgruppe aufweisen. Insbesondere handelt es sich bei den mehrfunktionellen Carbonsäuren um aromatische und/oder aliphatische Dicarbonsäuren bzw. deren Anhydride oder Ester.

Die zur Veresterung eingesetzten aliphatischen Dicarbonsäuren sollen mindestens 4 C-Atome aufweisen. Sie können linear oder verzweigt sein, wie z. B. Malonsäure, Bernsteinsäure, Fumarsäure, Dimethylglutarsäure oder Trimethyladipinsäure sowie deren Anhydride oder Ester.

Beispiele geeigneter aromatische Dicarbonsäuren sind Phthalsäure, Terephtalsäure, sowie Isophtalsäure und/oder deren Anhydride. Weiterhin ist Hexahydro-4-methylphtalsäureanhydrid geeignet.

Als mehrfunktionelle Carbonsäuren, insbesondere Dicarbonsäuren können auch sogenannte Dimerfettsäuren eingesetzt werden. Dabei handelt es sich bekanntlich um ein Gemisch aus acyclischen und cyclischen Dicarbonsäuren, die durch eine katalysierte Dimerisierung ungesättigter Fettsäuren mit 8 bis 22 C-Atomen erhalten werden. Zur Herstellung und Verwendung von Dimersäuren und deren physikalische und chemische Eigenschaften wird auf die Veröffentlichung "The Dimer Acids: The chemical und physical properties, reactions and applications", Ed. E.C. Leonard; Humko Sheffield Chemical, 1975, Memphis, Tenn., verwiesen.

Die oben genannten Dicarbonsäuren können auch in untergeordnetem Ausmaß tri- und mehrfunktionelle Carbonsäuren enthalten. Die Funktionalität des Gemisches sollte vorzugsweise im molaren Mittel einen Wert von 2,4 nicht überschreiten.

Erfindungsgemäß bevorzugte mehrfunktionelle Carbonsäuren sind DimerFettsäuren, Di- und Tricarbonsäuren, insbesondere Oxalsäure, Fumarsäure, Malonsäure, Bernsteinsäure, Glutarsäure, Adipinsäure, Pimelinsäure, Korksäure, Azelainsäure, Sebacinsäure, Dodecandisäure und/oder deren Anhydride oder Ester, wobei Dodecandisäure, Korksäure und Sebacinsäure und/oder deren Anhydride oder Ester besonders bevorzugt sind.

Die erfindungsgemäß eingesetzten bzw. erfindungsgemäßen Polyglycerinpartialester weisen insbesondere eine Polydispersität von mindestens 2, vorzugsweise von mindestens 5 und insbesondere bevorzugt von 3,5 bis 5 auf. Die bevorzugte Polydispersität deutet auf eine verzweigte bzw. hyperverzweigte Polymerstruktur hin. Diese verzweigte bzw. hyperverzweigte Polymerstruktur trägt zu den gewünschten Eigenschaften der erfindungsgemäß eingesetzten bzw. erfindungsgemäßen Polyglycerinester bei.

Die Bestimmung der Molmassenverteilung und damit der Polydispersität erfolgt im Rahmen der vorliegenden Erfindung nach Norm DIN 55672-1. In dieser Norm wird das Verfahren und die Prüfbedingungen zur Ermittlung der Molmassenverteilung und der Molmassenmittelwerte $M_n$ (Zahlenmittel) und $M_w$ (Gewichtsmittel) von Tetrahydrofuran-(THF-)löslichen Polymeren durch Gelpermeationschromatographie (GPC) beschrieben. Bei dem beschriebenen Verfahren handelt es sich nicht um ein Absolutverfahren. Vielmehr ist eine Kalibrierung, die mit kommerziell verfügbaren, linear aufgebauten und nach unabhängigen Absolutverfahren charakterisierten Polystyrol-Standards durchgeführt wird, notwendig.

Die erfindungsgemäßen Polyglycerinpartialester wurden im Rahmen der vorliegenden Erfindung wie folgt charakterisiert:

Gerät: Agilent 1100 von Agilent Technologie
Säulenkombination: SDV 1000/10000Å, Länge 65,00 cm, Temperatur 30 °C, THF als Mobile Phase, Fließrate 1ml/min, Probenkonzentration 10 g/l. RI Detektor,
Auswertung gegen Polystyrolstandard von 162 - 2520000 g/mol.

Die erfindungsgemäß eingesetzten bzw. erfindungsgemäßen Polyglycerinpartialester weisen weiterhin bevorzugt eine OH-Zahl von 10 - 160 mg/KOH, insbesondere 13-14 mg/KOH auf.

Die Hydroxylzahl wird im Rahmen der vorliegenden Erfindung in Anlehnung an DGF C-V 17 a (53), Ph. Eur. 2.5.3. Method A bestimmt.

Hierbei gibt die Hydroxylzahl an, wie viel mg Kaliumhydroxid der von 1 g der untersuchten Substanz bei der Acetylierung gebundenen Essigsäure äquivalent sind.

Zur Bestimmung wird die Probe mit Essigsäureanhydrid in Gegenwart von Pyridin acetyliert. Pro Mol an Hydroxylgruppe entsteht hierdurch ein Mol an Essigsäure, während das überschüssige Essigsäureanhydrid pro Mol Essigsäureanhydrid zwei Mol Essigsäure entstehen lässt.

Der Verbrauch an Essigsäure wird mittels Titration aus der Differenz zwischen Haupt- und einem parallel durchzuführenden Blindwert ermittelt bzw. berechnet.

Zur Bestimmung werden die folgenden Reagenzien benötigt.
Pyridin z.A. (z. B. Baker Art.-Nr. 8073)
Essigsäureanhydrid > 95 % (z. B. Baker Art.-Nr. 6068)
Für die Bestimmung wird eine Mischung der beiden Reagenzien hergestellt. Die Mischung besteht hierbei aus 23 % Essigsäureanhydrid und 77 % Pyridin. Die Mischung wird in einer getönten Glasflasche hergestellt. Die Lösung wird vor Gebrauch 24h gerührt.
Phenolphthalein - Lösung (z.B. Merck Art.-Nr. 7233.0100), 1% ig in Ethanol
Kalilauge 0,5 mol/l in Ethanol
Ethanol techn. > 98 %
Ethanol tech. > 98 %; neutralisiert gegen Phenolphtalein mittels ethanolischer
Kalilauge (0,5 mol/l)
dest. Wasser
Zur Erreichung einer akzeptablen Analysengenauigkeit sind die Probeneinwaage und die verwendete Menge des Acetylierungsgemisches so zu wählen, dass auf ein Mol Hydroxylgruppen 4 Mol Essigsäureanhydrid kommen. In einem 250 ml Rundkolben wird die der vermuteten Hydroxylzahl entsprechende Probenmenge auf + / - 0,1 mg genau auf einer Analysenwaage eingewogen.
Die Größe der Einwaage und das erforderliche Volumen des Acetylierungsgemisches, das genau abgemessen zuzufügen ist, kann aus der folgenden Tabelle ersehen werden.

| Erwartete OHZ | Acetylierungs-Gemisch in ml | Einwaage in g |
|---|---|---|
| 10 - 100 | 5 | 2,00 |
| 100-150 | 5 | 1,50 |
| 150-200 | 5 | 1,00 |
| 200 - 250 | 5 | 0,75 |
| 250 - 300 | 5 oder<br>10 | 0,60<br>1,20 |
| 300 - 350 | 10 | 1,00 |
| bis 700 | 15 | 0,75 |
| bis 950 | 15 | 0,50 |
| bis 1500 | 15 | 0,30 |
| bis 2000 | 15 | 0,20 |

Unter Rückflusskochen wird der mit Probenmaterial und Lösungsmittel befüllte Kolben auf 95 - 100 °C erhitzt. Nach 60 min Temperierung wird 1 ml dest. Wasser zugesetzt. Nach weiteren 10 min wird der Kolben aus dem Temperierbad genommen und unter Zuhilfenahme eines Wasserbades auf Raumtemperatur abgekühlt.
Am Kolbenhals kondensierte Flüssigkeit wird mit 5 ml neutralisiertem Alkohol in den Kolben überführt. Im Anschluss kann die Titration mit 0,5 n Kalilauge erfolgen.
Der zur Berechnung ebenfalls benötigte Blindversuch wird gesondert nach obiger Beschreibung durchgeführt, mit Ausnahme der Probenzugabe.
Die Bestimmung der Säurezahl der untersuchten Probe ist ebenfalls notwendig.
Unter Berücksichtigung des verbrauchten Volumens an 0,5 n Kalilauge im Haupt- und Blindversuch sowie der Säurezahl der Probe und der Einwaage, wird die Hydroxylzahl errechnet.
Die Berechnung der Hydroxylzahl erfolgt nach folgender Gleichung

$$OHZ = \frac{(b-a) \bullet 56{,}16\,g/mol \bullet N_{KOH}}{E} + SZ$$

a = verbrauchte ml 0,5 n Kalilauge im Hauptversuch
b = verbrauchte ml 0,5 n Kalilauge im Blindversuch
E = Einwaage in g
$N_{KOH}$=Normalität der KOH Lösung

Die erfindungsgemäß eingesetzten bzw. erfindungsgemäßen Polyglycerinpartialester weisen weiterhin bevorzugt eine Säurezahl von 0,5 - 14 mg KOH/g, insbesondere von 10 - 11 mg KOH/g auf.

Die im Rahmen der vorliegenden Erfindung verwendete Methode zur Bestimmung der Säurezahl ist bereits vorab bei der Beschreibung des Veresterungsgrades beschrieben.

Die erfindungsgemäß eingesetzten bzw. erfindungsgemäßen Polyglycerinpartialester können in an sich bekannter Weise durch Erhitzen der Reaktionskomponenten und destillativer Abtrennung des entstehenden Reaktionswassers hergestellt werden. Zur Beschleunigung können saure oder basische Katalysatoren, wie Sulfonsäuren, Phosphorsäure oder phosphorige Säure, Lewissäuren, wie Zinnsalze, Alkali- oder Erdalkalimetalloxide oder -hydroxide, -alkoholate oder -salze eingesetzt werden. Der Zusatz eines Katalysators ist aber nicht unbedingt notwendig. Die Polyglycerinpartialester werden bevorzugt in einem einstufigen Verfahren hergestellt. Dazu werden das Polyglycerin, die gesättigten oder ungesättigten, linearen oder verzweigten Fettsäuren und/oder aromatischen Monocarbonsäuren und die mehrfunktionellen Carbonsäuren miteinander umgesetzt. Der fortschreitende Umsatz kann z. B. über das abgetrennte Reaktionswasser, durch Messung der Säurezahl oder durch Infrarotspektroskopie verfolgt werden.

Der Anteil des Polyglycerins bei der Umsetzung beträgt insbesondere 14 bis 30 Gew.%, vorzugsweise 15 bis 18 Gew.%, bezogen auf die Gesamtmischung.

Der Anteil der gesättigten oder ungesättigten, linearen oder verzweigten Fettsäuren und/oder aromatischen Monocarbonsäuren bei der Umsetzung beträgt insbesondere 35 bis 75 Gew.%, vorzugsweise 65 bis 72 Gew.-%, bezogen auf die Gesamtmischung.

Der Anteil der mehrfunktionellen Carbonsäuren bei der Umsetzung beträgt insbesondere 5 bis 40 Gew.%, vorzugsweise 8 bis 12 Gew.%, bezogen auf die Gesamtmischung.

Die Umsetzung erfolgt insbesondere bei Temperaturen im Bereich von 200 bis 280 °C, insbesondere im Bereich von 230 bis 250 °C.

Die vorab beschriebenen Polyglycerinpartialester eignen sich bereits in vorteilhafter Weise für den Einsatz als Entschäumer, insbesondere in Lack- und Farbanwendungen.

Somit sind Entschäumer enthaltend Polyglycerinpartialester von gesättigten oder ungesättigten, linearen oder verzweigten Fettsäuren und/oder aromatischen Monocarbonsäuren und mehrfunktionellen Carbonsäuren, die erhältlich sind durch Veresterung eines Polyglyceringemisches mit gesättigten oder ungesättigten, linearen oder verzweigten Fettsäuren mit 4 bis 22 C-Atomen und/oder aromatischen Monocarbonsäuren mit 7 bis 22 C-Atomen und mehrfunktionellen Carbonsäuren mit 4 bis 54 C-Atomen und/oder deren Anhydride oder Ester, ebenfalls Gegenstand der vorliegenden Erfindung.

Darüber hinaus kann die entschäumende Eigenschaft der erfindungsgemäß eingesetzten bzw. erfindungsgemäßen Polyglycerinpartialester durch die Kombination mit Polyethern und oder freien Fettsäuren weiter verstärkt werden, das heißt in einer weiteren bevorzugten Ausführungsform enthalten die erfindungsgemäßen Entschäumer zusätzlich einen oder mehrere Polyether und/oder eine oder mehrere freie Fettsäuren.

Beispiele geeigneter Polyether sind unter Einsatz von Ethylenoxid und oder Propylenoxid als Ausgangstoffe hergestellte Moleküle, die als Startalkohol auf Butanol, Propylenglykol oder Allylalkohol zurückgreifen. Sofern Allylalkoholgestarteter Polyether verwendet werden, kommen auch Polyether in Frage, deren endständige Hydroxyfunktion mit Methanol umgesetzt/verkappt wurden.

Insbesondere bevorzugt sind Butanol-gestartete Polyether, bei deren Herstellung von den in Frage kommenden Epoxiden Ethylenoxid und Propylenoxid vorzugsweise Propylenoxid verwendet wurde.

Als freie Fettsäuren eignen sich Ölsäure, Isostearinsäure, Ricinolsäure und Linolsäure wobei vorzugsweise Ölsäure und oder Isostearinsäure eingesetzt wird.

Die hierbei erhaltenen physikalischen Mischungen bestehen vorzugsweise aus 20 - 80 Gew.% des Polyglycerinpartialesters, 20 - 80 Gew.% eines Polyethers sowie gegebenenfalls aus 2 - 15 Gew.-% eine oder mehrerer freier Fettsäuren.

Eine vorteilhafte entschäumende Wirkung kann in verschiedenen Lack- und Farbanwendungen beobachtet werden. Zum Beispiel ist die vorteilhafte entschäumende Wirkung in auf Acrylatoligomeren beruhenden Lacksystemen festzustellen.

Insbesondere können auf Polyesteracrylat-, Urethanacrylat-, Epoxyacrylatoligomeren sowie Amino-modifizierte Oligoetheracrylate beruhende Lacksysteme genannt werden, die ihre Anwendung in strahlenhärtenden Anwendungen finden.

Durch die bei der Applikation dieser Systeme üblichen geringen Trocknungszeiten von nur weinigen Sekunden, muss ein geeigneter Entschäumer eine sehr schnelle Reduzierung des bei der Verarbeitung auftretenden Schaums zeigen.

Gleichzeitig dürfen nur wenige Oberflächenstörungen durch den eingesetzten Entschäumer auftreten.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiele:

Die Bestimmung der in den Beispielen genannten Parameter erfolgt nach den bei der Beschreibung der Erfindung genannten Methoden.

Beispiel 1

Umsetzungsprodukt aus Polyglyzerin (3), Isostearinsäure und Sebazinsäure im stöchiometrischen Verhältnis 1 mol zu 3,5 mol zu 0,75 mol.

Die Ausgangsstoffe wurden im stöchiometrichen Verhältnis Polyglycerin (3) 1 mol zu Isostearinsäure 3,5 mol zu Sebazinsäure 0,75 mol in einer Reaktionsapparatur vorgelegt und unter Rühren auf 240 °C erhitzt. Diese Temperatur wurde unter Rühren gehalten bis eine Säurezahl von < 12 mg KOH / g erreicht war.

Im Anschluss wurde das Reaktionsgemisch unter Rühren abgekühlt und abgefüllt.

Ermittelte analytische Kenndaten:

Veresterungsgrad laut Säurezahl: 94,5 %

OH - Zahl: 14 mg/g

Säurezahl: 11,0 mg/g

Mw: 10814 g/mol

Mn: 2769 g/mol

Polydispersität: 3,9

Auf die in Beispiel 1 beschriebene Art und Weise wurden auch die Beispiele 2 - 6 hergestellt.

| **Beispiel Nr.** | 2 | 3 | 4 | 5 | 6 |
|---|---|---|---|---|---|
| | | | | | |
| **Rohstoff Mengen (mol)** | | | | | |
| Polyglycerin - 3 | 1 mol | 1 mol | 1 mol | 1 mol | 1 mol |
| Isostearingsäure | 1,5 mol | 3,25 mol | 3,25 mol | 3,5 mol | 1,5 mol |
| Dimersäure (CAS Nummer 61788-89-4) | 0,75 mol | 0,375 mol | - | 0,75 mol | - |
| Sebazinsäure | - | - | 0,375 mol | - | 0,75 mol |
| | | | | | |
| **Kenndaten** | | | | | |
| SZ [mg KOH/g] | 2,5 | 3,1 | 3 | 5,4 | 0,9 |
| Umsatz laut SZ in % bezogen auf die eingesetzten Carboxylfunktionalitäten | 98,4 | 98,1 | 98,3 | 96,7 | 99,6 |
| Polydispersität (Mw/Mn) | 11,8 | 2,02 | 1,79 | 5,5 | 9,46 |
| Mw [g/mol] | 44335 | 4964 | 3757 | 23431 | 26525 |
| Mn [g/mol] | 3756 | 2467 | 2099 | 4266 | 2804 |
| OH Zahl [mg KOH/g] | 111,0 | 46 | 47 | 11,0 | 145 |

Exemplarisch gefundene anwendungstechnische Ergebnisse zu den aufgeführten Beispielen:

Zur Bestimmung der anwendungstechnischen Eigenschaften wurde Luft in 50 g einer Acrylatoligomermischung (z. B. bestehend aus aliphatischen Urethantriacrylaten (Ebecryl 265), Polyesteracrylaten (Ebecryl 452) oder Epoxyacrylaten (Laromer 8986) mittels eines handelsüblichen Labordissolvers (1 min / 3000 upm) eingerührt. Im Falle der erfindungsgemäßen Beispiele wurden 0,50 g der erfindungsgemäßen Polyglycerinpartialester der Acrylatoligomermischung zugefügt.

Die Schaumhöhe nach Belastung steht für die bei Einrühren von Luft entstandene Schaumhöhe.

Die Zeit bis zur Schaumfreiheit steht für die Zeitdauer bis zum vollständigen Zusammenbruch des entstandenen Schaums.

Die Beurteilung der Verträglichkeit steht für die Beeinflussung der Oberflächenqualität. Diese wurde in einer 10-er Skala beurteilt. Der Wert 10 entspricht hierbei keinerlei Beeinflussung, und der Wert 1 für eine sehr starke negative Beeinflussung.

| Beispiel Nr. | Nullprobe ohne Zusatz an Entschäumer | Beispiel 1 | Beispiel 2 | Beispiel 3 | Beispiel 4 | Beispiel 5 | Beispiel 6 |
|---|---|---|---|---|---|---|---|
| | | | | | | | |
| Schaumhöhe nach Belastung [mm] | 4 | 1 | 2 | 1 | 1 | 2 | 2 |
| Zeit bis Schaumfreiheit [min] | 80 | 55 | 60 | 62 | 60 | 55 | 60 |
| Verträglichkeit | 10 | 8 | 7 | 7 | 8 | 8 | 7 |

Die anwendungstechnischen Beispiele zeigen, dass durch erfindungsgemäße Verwendung der Polyglycerinpartialester als Entschäumer die Schaumhöhe und die Zeit bis zur Schaumfreiheit gegenüber Systemen ohne Entschäumer deutlich reduziert werden kann, ohne die Verträglichkeit stark zu verschlechtern.

**Patentansprüche**

1. Verwendung von Polyglycerinpartialestern von gesättigten oder ungesättigten, linearen oder verzweigten Fettsäuren und/oder aromatischen Monocarbonsäuren und mehrfunktionellen Carbonsäuren, die erhältlich sind durch Veresterung eines Polyglyceringemisches mit gesättigten oder ungesättigten, linearen oder verzweigten Fettsäuren mit 4 bis 22 C-Atomen und/oder aromatischen Monocarbonsäuren mit 7 bis 22 C-Atomen und mehrfunktionellen Carbonsäuren mit 4 bis 54 C-Atomen und/oder deren Anhydride oder Ester als Entschäumer, **dadurch gekennzeichnet, dass** der Veresterungsgrad des Polyglycerins zwischen 76 und 100 % liegt, und dass die Polyglyceringemische auf Polyglycerinen mit einem mittleren Kondensationsgrad von 3-5 basieren.

2. Verwendung gemäß Anspruch 1, **dadurch gekennzeichnet, dass** die gesättigte oder ungesättigte, lineare oder verzweigte Fettsäure mit 4 bis 22 C-Atomen ausgewählt ist aus Isostearinsäure, Ölsäure und/oder Linolensäure.

3. Verwendung gemäß Anspruch 1 oder 2, **dadurch gekennzeichnet, dass** die mehrfunktionellen Carbonsäuren ausgewählt sind aus Oxalsäure, Fumarsäure, Malonsäure, Bernsteinsäure, Glutarsäure, Adipinsäure, Pimelinsäure, Korksäure, Azelainsäure, Sebacinsäure, Dodecandisäure, Phthalsäure und/oder deren Anhydride oder Ester.

4. Verwendung gemäß einem oder mehreren der Ansprüche 1 bis 3, **dadurch gekennzeichnet, dass** die Polyglycerinpartialester eine Polydispersität von mindestens 2 aufweisen.

5. Entschäumer enthaltend Polyglycerinpartialester von gesättigten oder ungesättigten, linearen oder verzweigten Fettsäuren und/oder aromatischen Monocarbonsäuren und mehrfunktionellen Carbonsäuren, die erhältlich sind durch Veresterung eines Polyglyceringemisches mit gesättigten oder ungesättigten, linearen oder verzweigten Fettsäuren mit 4 bis 22 C-Atomen und/oder aromatischen Monocarbonsäuren mit 7 bis 22 C-Atomen und mehrfunktionellen Carbonsäuren mit 4 bis 54 C-Atomen und/oder deren Anhydride oder Ester, wobei der Veresterungsgrad des Polyglycerins zwischen 91 und 100 % liegt, und wobei die Polyglyceringemische auf Polyglycerinen mit einem mittleren Kondensationsgrad von 3-5 basieren.

6. Entschäumer gemäß Anspruch 5, **dadurch gekennzeichnet, dass** sie zusätzlich einen oder mehrere Polyether und/oder eine oder mehrere freie Fettsäuren enthalten.

**Claims**

1. Use of polyglycerol partial esters of saturated or unsaturated, linear or branched fatty acids and/or aromatic monocarboxylic acids and polyfunctional carboxylic acids, which are available by esterification of a polyglycerol mixture with saturated or unsaturated, linear or branched fatty acids having 4 to 22 carbon atoms and/or aromatic monocarboxylic acids having 7 to 22 carbon atoms and polyfunctional carboxylic acids having 4 to 54 carbon atoms and/or their anhydrides or esters as defoamers, **characterized in that** the degree of esterification of the polyglycerol is between 76 and 100%, and **in that** the polyglycerol mixtures are based on polyglycerols with an average degree of condensation of 3-5.

2. Use according to Claim 1, **characterized in that** the saturated or unsaturated, linear or branched fatty acid having 4 to 22 carbon atoms is selected from isostearic acid, oleic acid and/or linolenic acid.

3. Use according to Claim 1 or 2, **characterized in that** the polyfunctional carboxylic acids are selected from oxalic acid, fumaric acid, malonic acid, succinic acid, glutaric acid, adipic acid, pimelic acid, suberic acid, azelaic acid, sebacic acid, dodecanedioic acid, phthalic acid and/or their anhydrides or esters.

4. Use according to one or more of Claims 1 to 3, **characterized in that** the polyglycerol partial esters have a polydispersity of at least 2.

5. Defoamers comprising polyglycerol partial esters of saturated or unsaturated, linear or branched fatty acids and/or aromatic monocarboxylic acids and polyfunctional carboxylic acids, which are obtainable by esterification of a polyglycerol mixture with saturated or unsaturated, linear or branched fatty acids having 4 to 22 carbon atoms and/or aromatic monocarboxylic acids having 7 to 22 carbon atoms and polyfunctional carboxylic acids having 4 to 54 carbon atoms and/or their anhydrides or esters, wherein the degree of esterification of the polyglycerol is between 91 and 100%, and wherein the polyglycerol mixtures are based on polyglycerols with an average degree of condensation of 3 - 5.

6. Defoamers according to Claim 5, **characterized in that** they additionally comprise one or more polyethers and/or one or more free fatty acids.

**Revendications**

1. Utilisation d'esters partiels de polyglycérol d'acides gras saturés ou insaturés, linéaires ou ramifiés et/ou d'acides monocarboxyliques aromatiques et d'acides carboxyliques polyfonctionnels, qui peuvent être obtenus par estérification d'un mélange de polyglycérols avec des acides gras saturés ou insaturés, linéaires ou ramifiés, comprenant 4 à 22 atomes de carbone et/ou des acides monocarboxyliques aromatiques comprenant 7 à 22 atomes de carbone et des acides carboxyliques polyfonctionnels comprenant 4 à 54 atomes de carbone et/ou leurs anhydrides ou esters comme antimousses, **caractérisée en ce que** le degré d'estérification du polyglycérol est situé entre 76 et 100% et **en ce que** les mélanges de polyglycérols sont à base de polyglycérols présentant un degré de condensation moyen de 3-5.

2. Utilisation selon la revendication 1, **caractérisée en ce que** l'acide gras saturé ou insaturé, linéaire ou ramifié, comprenant 4 à 22 atomes de carbone est choisi parmi l'acide isostéarique, l'acide oléique et/ou l'acide linolénique.

3. Utilisation selon la revendication 1 ou 2, **caractérisée en ce que** les acides carboxyliques polyfonctionnels sont choisis parmi l'acide oxalique, l'acide fumarique, l'acide malonique, l'acide succinique, l'acide glutarique, l'acide adipique, l'acide pimélique, l'acide subérique, l'acide azélaïque, l'acide sébacique, l'acide dodécanedioïque, l'acide phtalique et/ou leurs anhydrides ou esters.

4. Utilisation selon l'une ou plusieurs des revendications 1 à 3, **caractérisée en ce que** les esters partiels de polyglycérol présentent une polydispersité d'au moins 2.

5. Antimousses contenant des esters partiels de polyglycérol d'acides gras saturés ou insaturés, linéaires ou ramifiés et/ou d'acides monocarboxyliques aromatiques et d'acides carboxyliques polyfonctionnels, qui peuvent être obtenus par estérification d'un mélange de polyglycérols avec des acides gras saturés ou insaturés, linéaires ou ramifiés, comprenant 4 à 22 atomes de carbone et/ou des acides monocarboxyliques aromatiques comprenant 7 à 22 atomes de carbone et des acides carboxyliques polyfonctionnels comprenant 4 à 54 atomes de carbone et/ou leurs anhydrides ou esters, le degré d'estérification du polyglycérol se situant entre 91 et 100% et les mélanges de polyglycérols étant à base de polyglycérols présentant un degré de condensation moyen de 3-5.

6. Antimousses selon la revendication 5, **caractérisés en ce qu'**ils contiennent en outre un ou plusieurs polyéthers et/ou un ou plusieurs acides gras libres.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6605183 B
- US 5914362 A
- US 5846454 A
- US 4690713 A
- US 4976888 A
- US 5429718 A
- US 5346511 A
- DE 4404202
- EP 07655811 A
- EP 450605 A

- WO 00051708 A
- DE 2517354
- JP 2000230191 A
- EP 0403913 A1
- JP 2011083715 A
- EP 0878224 A
- DE 19641076 A1
- DE 19835968 A1
- WO 2011100420 A1
- EP 0835862 A

**In der Beschreibung aufgeführte Nicht-Patentliteratur**

- **CASSEL et al.** Original synthesis of linear, branched and cyclic oligoglycerol standards. *Eur. J. Org. Chem.,* 2001, 875-896
- **THIELE R et al.** *J Agric Food Chem,* 2008, vol. 56, 4219

- **SPENCER GF et al.** *Lipids,* 1979, vol. 14, 72
- The Dimer Acids: The chemical und physical properties, reactions and applications. Humko Sheffield Chemical. Memphis, Tenn, 1975